Europäisches Patentamt

European Patent Office

Office européen des brevets

(19)

(11) Numéro de publication: **0 254 614**
**A1**

(12) **DEMANDE DE BREVET EUROPEEN**

(21) Numéro de dépôt: **87401506.8**

(22) Date de dépôt: **30.06.87**

(51) Int. Cl.4: **C 12 N 15/00**
A 61 K 37/64, C 12 P 21/02

(30) Priorité: **02.07.86 FR 8609608**

(43) Date de publication de la demande:
**27.01.88 Bulletin 88/04**

(84) Etats contractants désignés:
**AT BE CH DE ES FR GB GR IT LI LU NL SE**

(71) Demandeur: **TRANSGENE S.A.**
**16, rue Henri Régnault**
**F-92400 Courbevoie (FR)**

(72) Inventeur: **Skern, Tim**
**15 rue de Saint-Dié**
**F-67100 Strasbourg (FR)**

**Jallat, Sophie**
**8, quai Mathis**
**F-67000 Strasbourg (FR)**

**Courtney, Michael**
**11, rue des Lilas Geudertheim**
**F-67170 Brumath (FR)**

(74) Mandataire: **Warcoin, Jacques et al**
**Cabinet Régimbeau 26, avenue Kléber**
**F-75116 Paris (FR)**

(54) **Variants de l'alpha-antitrypsine humaine glycosylée et procédé pour la préparation.**

(57) La présente invention concerne un vecteur viral d'expression de l'$alpha_1$-AT ou d'un variant de l'$alpha_1$-AT choisi parmi : [AA$^{357}$]alpha$_1$-AT, [AA$^{358}$]alpha$_1$-AT [AA$^{357}$, AA$^{358}$] alpha$_1$-AT et les variants correspondants de la forme tronquée [ delta 1→5 ]alpha$_1$-AT ainsi que [ delta 1→5 ] alpha$_1$-AT, caractérisé en ce qu'il est constitué d'un virus hybride de la vaccine qui comporte une séquence d'ADN codant pour ledit variant de l'alpha$_1$-AT inséré dans une partie non essentielle du génome du virus et sous le contrôle d'une séquence d'expression efficace dans la vaccine.

EP 0 254 614 A1

## Description

VARIANTS DE L'ALPHA-ANTITRYPSINE HUMAINE GLYCOSYLEE ET PROCEDE POUR LA PREPARATION.

L'alpha$_1$-antitrypsine (alpha$_1$-AT) est l'inhibiteur de protéase le plus abondant dans le plasma.

L'inhibition de cette protéase sérine joue un rôle important dans le contrôle de l'élastase neutrophile humaine et du facteur XIa . Le centre réactif de l'alpha$_1$-AT est formé par les deux résidus amino-acides appelés P$_1$ et P'$_1$. La nature du résidu P1 détermine la spécificité de l'inhibition par l'alpha$_1$-AT et le remplacement de la méthionine P1 naturelle par l'arginine (alpha$_1$-AT Met$^{358}$ → Arg$^{358}$) produit un inhibiteur qui présente une affinité largement augmentée pour la thrombine avec une réduction concomitante dans l'affinité pour l'élastase neutrophile humaine.

Le variant alpha$_1$-AT(Met$^{358}$ → Arg$^{358}$) est également capable d'inhiber efficacement des enzymes de phase de contact de la protéolyse du plasma, ceci en contraste avec la protéine native. L'inhibition de la kallikréine et du facteur XIa est augmentée entre 20 000 et 70 000 fois respectivement par la présence de l'arginine en position 358. Le facteur XII activé (XIIf) est également inhibé efficacement, l'alpha$_1$-AT native ne présente aucune activité à l'égard de cette protéase. Comme les protéases du système de phase de contact sont activées dans les maladies telles que les chocs septiques, les attaques d'angio-eodème héréditaire et les syndromes de détresse respiratoire des adultes, l'administra-tion de l'alpha$_1$-AT (Met$^{358}$ → Arg$^{358}$) peut être très efficace dans le contrôle de ces maladies.

L'alpha$_1$-AT qui se trouve dans le plasma est glycosylée. Il est donc souhaitable que l'alpha$_1$-AT (Met$^{358}$ → Arg$^{358}$) produite pour utilisation in vivo soit également modifiée de cette façon.

De même, la Demanderesse ayant démontré l'intérêt d'utiliser d'autres variants de l'alpha$_1$ -AT, il est également intéressant de pouvoir préparer ces variantes sous forme glycosylée.

Il s'agit notamment des variants tronqués :
(delta Glu$^1$→ Gly$^5$) alpha$_1$-AT qui seront notés [delta 1→5] alpha$_1$-AT ainsi que des variants de alpha$_1$-AT ou de alpha$_1$-AT mutés en position 357 et/ou 358 notés [AA$^{357}$] alpha$_1$-AT [AA$^{358}$] alpha$_1$-AT ou [AA$^{357}$ AA$^{358}$] alpha$_1$AT.

En particulier, outre les variants Arg$^{358}$, les variants suivants sont intéressants comme cela est démontré dans le brevet WO 86 00337 :
(Gly$^{358}$), (Ala$^{358}$), (Ile$^{358}$), (Val$^{358}$), Leu$^{358}$) et (Phe$^{358}$) qui peuvent également être tronqués. En plus le variant [Lys $^{358}$] alpha$_1$-AT présente un intérêt comme inhibiteur spécifique de la plasmine.

Enfin, les variants en position 357 tels que Ala$^{357}$ sont également intéressants en particulier le variant [ Ala$^{357}$ , Arg$^{358}$ ] qui présentent des propriétés améliorées par rapport au variant Arg$^{358}$ décrit précédemment.

Ces variants sont préparés selon l'invention par culture de cellules eucaryotes en particulier des cellules de mammifères incorporant un bloc d'expression du gêne correspondant au variant désiré.

En particulier, ce bloc d'expression est situé sur vecteur viral, particulièrement un poxvirus tel que la vaccine. Il s'agit d'un vecteur viral d'expression de l'alpha$_1$-AT ou d'un variant de l'alpha$_1$-AT choisi parmi : [AA$^{357}$]alpha$_1$-AT, [AA$^{358}$]alpha$_1$-AT [AA$^{357}$, AA$^{358}$] alpha$_1$-AT et les variants correspondants de la forme tronquée [delta 1→5]alpha$_1$-AT ainsi que [delta 1→5]alpha$_1$-AT caractérisé en ce qu'il est constitué d'un virus hybride de la vaccine qui comporte une séquence d'ADN codant pour l'al-pha$_1$-AT ou ledit variant de l'alpha$_1$-AT inséré dans une partie non essentielle du génome du virus et sous le contrôle d'une séquence d'expression efficace dans la vaccine.

Ce type de construction a déjà été décrit par la Demanderesse notamment dans les brevets WO 85/04810 et WO 85/05376. L'expression et la modification post traductionnelle correcte de différentes protéines étrangères dans les cellules de mammifères, en utilisant le virus de la vaccine,ont été décrites à plusieurs reprises.

Pour ce faire, le gêne correspondant au variant dérivé de l'alpha$_1$-AT est inséré de préférence dans une partie du génome non essentielle du virus de la vaccine et de façon encore préférable dans un gêne marqueur tel que le gêne TK.

L'insertion dans ce gêne supprime son expression et les cellules transfectés par le virus correspondant, peuvent être sélectionnées comme cela sera décrit ci-après.

Le bloc d'expression comprendra de préférence une séquence assurant l'expression du gêne en cause dans les cellules après transfection, en particulier le gêne codant pour le variant de l'alpha$_1$-AT sera sous la dépendance d'un promoteur de la vaccine tel que le promoteur du gêne 7,5 K qui sera noté P 7,5 K.

Des cellules de mammifères, notamment des cellules BHK infectées par le vecteur virus hybride décrit précédemment produisent des variants de l'alpha$_1$-AT si elles sont cultivées sur des milieux de culture appropriés.

Les vecteurs en cause sont en général obtenus par co-transfection d'un virus vaccine sauvage et d'un plasmide portant les éléments évoqués précédemment, le virus hybride est constitué in vivo, par recombinaison, cette technologie est décrite dans les brevets cités précédemment.

L'invention concerne également l'alpha$_1$-AT et les variants de l'alpha$_1$-AT glycosylé ainsi obtenue, ces produits pouvant être séparés du milieu de culture par des procédés connus dans la technologie des protéines.

Il s'agit en particulier des variants
[ AA$^{357}$ ] alpha$_1$-AT
[ AA$^{358}$ ] alpha$_1$-AT
[ AA$^{357}$ AA$^{358}$ ] alpha$_1$-AT
et des variants [delta 1→5 ]alpha$_1$-AT correspon-dant ainsi que des variants [delta 1→5 ]alpha$_1$-AT, ces variants étant glycosylés et dépourvus de résidu Met à l'extrémité 3'. Parmi ces variants, il faut citer les variants :

[ Ala$^{357}$ Arg$^{358}$ ]alpha$_1$-AT glycosylé
[ delta 1→5 ] [Arg$^{358}$]alpha$_1$-AT glycosylé
[ delta 1→5 ] [Ala$^{357}$Arg$^{358}$ ] alpha$_1$-AT glycosylé, et
[Arg$^{358}$] alpha$_1$-AT glycosylé.

Ces variants de l'alpha$_1$-AT seront utiles comme médicaments en particulier pour le remplacement de l'alpha$_1$ AT obtenue par extraction à partir du plasma,et certains d'entre eux comme inhibiteurs de la kallikreine.

Les exemples suivants sont destinés plus particulièrement à mettre en évidence les constructions utilisables dans le cadre de la présente invention.

EXEMPLE 1 :

CONSTRUCTION DE L'ADN DE pTG1952

Le plasmide pTG1952 a la structure suivante :
A l'extrémité 5' du cADN de l'alpha$_1$-AT se trouve la séquence signal (ss), une séquence qui est responsable de l'exportation de la protéine vers l'extérieur de la cellule. Pendant la maturation de la protéine, cette séquence est clivée pour produire la protéine native. Cette construction contient également le codon arginine à l'amino-acide 358. Le clivage de ce plasmide par BglII produit un fragment de 1,3 kb contenant l'ensemble du cADN de l'alpha$_1$-AT et la séquence signal.

EXEMPLE 2

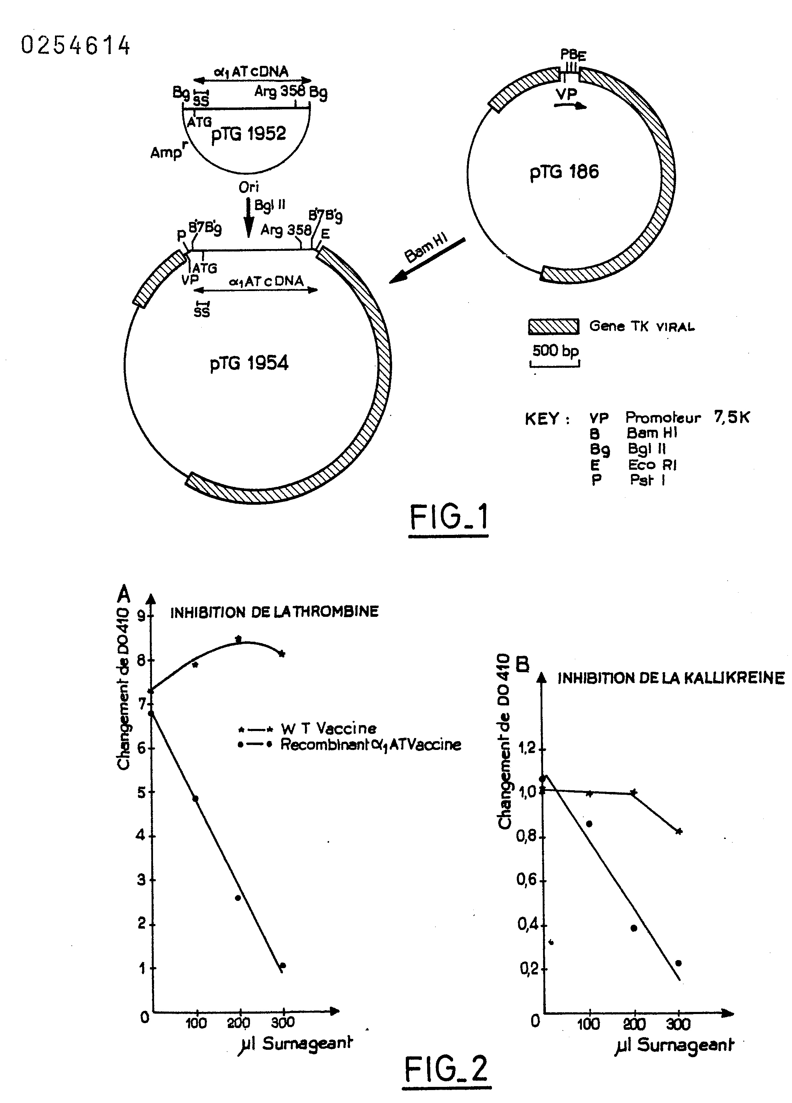

PREPARATION DE pTG1954 (figure 1)

Le plasmide pTG186 contient le gène codant pour le protéine de la thymidine kinase du virus de la vaccine. Un fragment d'ADN de petite taille contenant le promoteur 7,5 K du virus de la vaccine (VP) et un "polylinker" possédant différents sites de restriction dont BamHI ont été placés dans le gène de la thymidine kinase comme cela est indiqué ci-après.

Le fragment 1,3 kb BglII de pTG1952 est inséré dans le site BamHI de pTG186 pour produire le plasmide pTG1954. L'orientation du fragment BglII inséré est étudié en utilisant le site BamHI dans le cADN de alpha$_1$-AT et est telle que l'amino-acide terminal de cette séquence de cADN est proximale au promoteur viral VP.

EXEMPLE 3

TRANSFECTION DE pTG1954 DANS LE VIRUS DE LA VACCINE

La production et la sélection des virus de vaccine recombinants TK- en utilisant pTG1954 ont été effectuées en utilisant des techniques connues qui sont décrites notamment dans les brevets PCT FR 85/00096 et WO 85/05376. 8 plaques sont choisies et utilisées pour infecter des mono-couches de cellules BHK en culture tissulaire

L'ADN est préparé à partir des cellules infectées avec des recombinants du virus de la vaccine TK- par des techniques connues. Après restriction avec HindIII, l'ADN est soumis à l'électrophorèse sur un gel d'agarose à 0,8 %.L'ADN est transféré sur nitrocellulose et le gène de l'alpha$_1$-AT détecté avec avec des oligonucléotides spécifiques phosphorylés au $^{32}$P.

Les 8 virus recombinants TK- sont trouvés avoir intégré le cADN de l'alpha$_1$-AT dans le gène TK. L'un de ceux-ci est choisi pour plus ample étude.

EXEMPLE 4

ESSAI D'UN MILIEU APRES INFECTION DES CELLULES BHK AVEC UN RECOMBINANT VACCINE CONTENANT pTG1954

Inhibition de la thrombine

Après incubation à 37°C pendant 24 heures, le milieu est centrifugé pour éliminer les cellules et les débris cellulaires et des aliquotes sont testées pour leur capacité à inhiber la thrombine.

L'essai est effectué dans des cuvettes de 1 ml dans une solution contenant 150 mM NaCl, 100 mM Tris pH 8,0 et 0,1 % de PEG (tampon TB). La thrombine (240 mU) est incubée dans le tampon TB avec différentes quantités de milieu pendant 20 minutes à température ambiante. La quantité d'activité thrombinique demeurant est mesurée en utilisant du substrat (Chromozyme TH Boehrimser mannheim ; 100 nanomoles) et en mesurant le changement dans la densité optique à 410 nanomètres après 4 minutes. La courbe obtenue à partir d'une expérience typique est indiquée à la figure 2A. 50 % d'inhibition avec cette quantité de thrombine (240 mU) est obtenue avec 150 µl de milieu. La courbe 0-0 représente le virus recombinant et x-x le virus sauvage.

Inhibition de la kallikréine

L'inhibition de la kallikréine est effectuée de manière similaire. la Kallikréine (4 mU) est incubée avec différentes quantités de milieu pendant 30 minutes à température ambiante dans des cuvettes contenant du tampon kallikréine (0,1 M phosphate de sodium (pH 6,5) ; 0,15 M NaCl). Les essais de l'activité résiduelle de kallikréine sont effectués par addition de substrat (225 nanomoles) et les changements dans la densité optique à 410 nanomètres sont mesurés après 4 minutes. La courbe obtenue à partir d'une expérience typique est représentée à la figure 2B. 50 % d'inhibition avec cette quantité de kallikréine (4 mU) est obtenue avec 120 µl de milieu dans ce cas.

EXEMPLE 5

IMMUNOPRECIPITATION D'ALPHA$_1$-AT (ARGININE VARIANT) MARQUE AU $^{35}$S PRODUIT PAR DES CELLULES DE MAMMIFERES

Des mono-couches de cellules BHK sont infectées avec 0,2 pfu de virus vaccine recombinant puis incubées pendant 6 heures à 37°C dans 1 ml de milieu contenant 100 µCi de méthionine S$^{35}$. Un contrôle est effectué avec un virus vaccine sauvage. Le milieu collecté comme précédemment et soumis à un challenge avec anticorps anti-alpha$_1$ antitrypsine comme suit. A 1 ml de milieu on ajoute du tampon RIPA (1MKCl, 2 % Triton X-100, 100 mM Tris pH 8,0) et 5 µl d'anticorps alpha-antitrypsine (Dako Immunoglobulins, Danemark) et le mélange est incubé pendant 2 heures à 25°C.

50 µl de tampon RIPA contenant de 8 mg de protéine A sepharose pré-équilibré sont alors ajoutés et le mélange est incubé avec une agitation éventuelle pendant 1 heure à 25°C. La sépharose protéine A est alors collectée par centrifugation et le culot est lavé 5 fois avec 0,5 M KCl, 10 mM Tris-HCl pH 8,8 0,5 % Triton X-100 et 2 fois avec 2 x 10 mM Tris KCl pH 8,8. Les culots sont dissous dans 100 µl de tampon Laemmli et 20 µl sont chargés sur un gel de polyacrylamide à 10 % qui est ensuite autoradiographié après fluorographie.

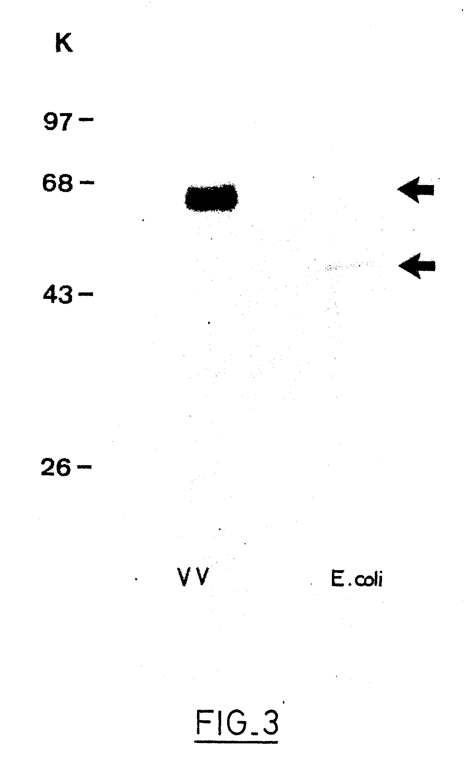

D'après un profil de gel typique (Figure 3) et en comparaison avec une immunoprécipitation utilisant l'antitrypsine-alpha₁ marquée méthionine obtenue à partir d'un extrait bactérien, on peut voir qu'une protéine est spécifiquement précipitée à partir du milieu de cellules infectées avec les virus vaccine recombinants. Cette protéine n'est pas présente dans les extraits cellulaires à partir de virus de la vaccine sauvage. Toutefois, cette protéine reconnue par les anticorps anti-alpha₁-AT est plus grande que celle qui est trouvée dans les extraits cellulaires bactériens, ceci constitue une indication que l'alpha₁-AT variant arginine produite dans les cellules de mammifères a été glycosylée.

EXEMPLE 6 :

En utilisant à la place du plasmide pTG1952, les plasmides décrits au brevet WO 86 00337, on obtient les variants correspondants glycosylés.

Par exemple pTG1981 qui est identique à pTG 1954, sauf que le gène introduit à [ Ala³⁵⁷ - Arg³⁵⁸ ] alpha₁-AT a été construit de la même façon.

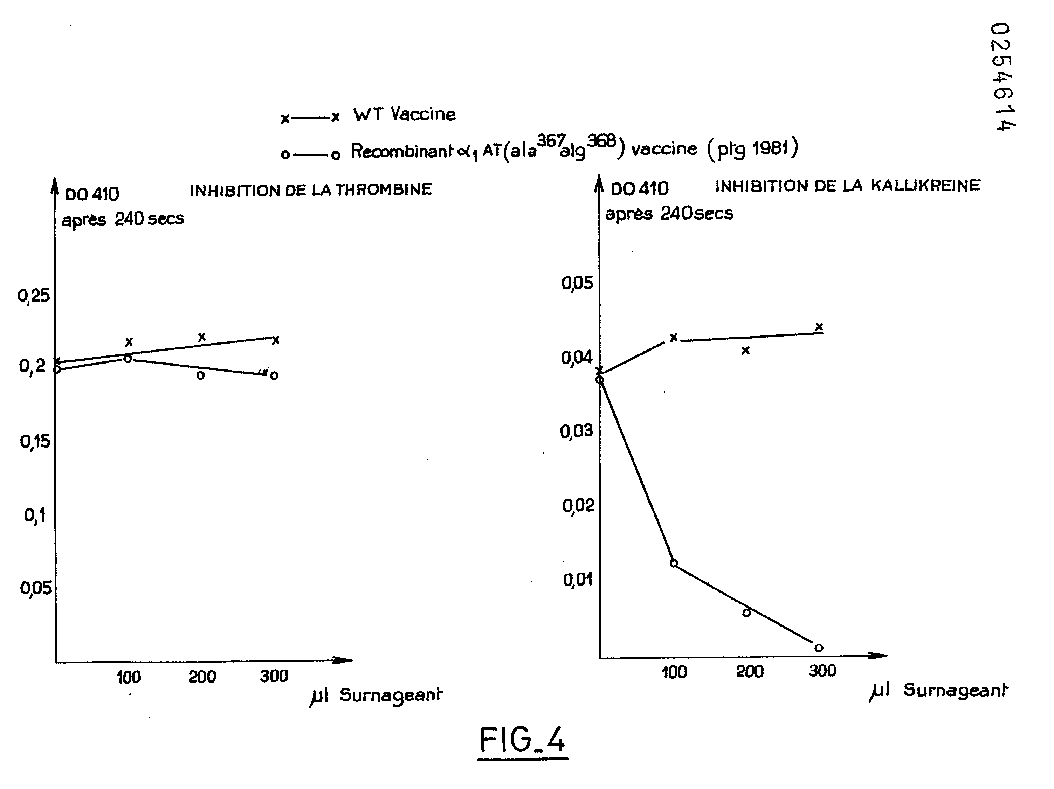

Les résultats des essais d'inhibition de la thrombine et de la Kallikréine pour le surnageant ont été représentés à la figure 4. Comme cela a été observé avec [ Ala³⁵⁷ - Arg³⁵⁸ ]alpha₁-AT produit par E. coli, le produit secrété à partir de cellules BHK est activé sur la Kallikréine mais a peu ou pas d'activité inhibitrice à l'égard de la thrombine.

**Revendications**

1. Vecteur viral d'expression de l'alpha₁-AT ou d'un variant de l'alpha₁-AT choisi parmi : [AA³⁵⁷]alpha₁-AT, [ AA³⁵⁸ ]alpha₁-AT [ AA³⁵⁷, AA³⁵⁸ ]alpha₁-AT et les variants correspondants de la forme tronquée [delta 1→5 ] alpha₁-AT ainsi que [ delta 1→5 ]alpha₁-AT, caractérisé en ce qu'il est constitué d'un virus hybride de la vaccine qui comporte une séquence d'ADN codant pour ledit variant de l'alpha₁-AT inséré dans une partie non essentielle du génome du virus et sous le contrôle d'une séquence d'expression efficace dans la vaccine.

2. Vecteur viral d'expression selon la revendication 1, caractérisé en ce que la séquence d'ADN codant pour l'alpha₁-AT ou pour ledit variant est précédé d'au moins une partie de la séquence signal.

3. Vecteur viral d'expression selon les revendications 1 et 2, caractérisé en ce que la séquence d'ADN est insérée dans le gène TK.

4. Vecteur viral d'expression selon les revendications 1 à 3, caractérisé en ce que la séquence d'expression comporte au moins un promoteur de la vaccine.

5. Vecteur viral d'expression selon la revendication 4, caractérisé en ce que le promoteur est le promoteur du gène 7,5 K.

6. Cellules de mammifères transfectées par un vecteur viral selon l'une des revendications 1 à 5.

7. Cellules selon la revendication 6, caractérisé en ce qu'il s'agit de cellules BHK.

8. Procédé de préparation de variants de l'alpha₁-AT, caractérisé en ce qu'on cultive des cellules transfectées selon l'une des revendications 6 et 7 sur un milieu approprié et l'on récupère le variant de l'alpha₁-AT obtenu.

9. Variants de l'alpha₁-AT
[ AA³⁵⁷ ] alpha₁-AT
[ AA³⁵⁸ ] alpha₁-AT
[ AA³⁵⁷ AA³⁵⁸ ] alpha₁-AT
et les variants de [delta 1→5 ] alpha₁-AT correspondants ainsi que le variant [ delta 1→5 ]alpha₁-AT, ces variants étant sous forme glycosylée.

10. Variants selon la revendication 9
[ Ala³⁵⁷ Arg³⁵⁸ ]alpha₁-AT glycosylé
[ delta 1→5 ] [Arg³⁵⁸ ]alpha₁-AT glycosylé
[ delta 1→5 ] [Ala³⁵⁷ Arg³⁵⁸ ] alpha₁-AT glycosylé.
+ [Arg³⁵⁸ ]-alpha₁-AT glycosylé.

11. A titre d'inhibiteur de la kallikreine un variant selon la revendication 10.

12. A titre de médicament un variant selon l'une des revendications 9 à 11.

0254614

FIG_1

FIG_2

0254614

FIG.3

FIG_4

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | THE NEW ENGLAND JOURNAL OF MEDICINE, vol. 309, no. 12, septembre 1983, pages 694-698; M.C. OWEN et al.: "Mutation of antitrypsin to antithrombin: alpha1-antitrypsin Pittsburgh (358 Met-Arg), a fatal bleeding disorder" * En entier * | 9-12 | C 12 N 15/00 A 61 K 37/64 C 12 P 21/02 |
| X,Y | TRENDS IN BIOTECHNOLOGY, vol. 3, no. 2, 1985, pages 51-53; P.W. BERMAN et al.: "Engineering glycoproteins for use as pharmaceuticals" * Page 52, colonnes 1-2; page 53, colonne 2 * | 1-12 | |
| Y | EP-A-0 114 777 (TRANSGENE) * Page 50, ligne 19 - page 51, ligne 6 * | 1-12 | DOMAINES TECHNIQUES RECHERCHES (Int. Cl.4) C 12 N A 61 K C 12 P |
| Y | EP-A-0 169 114 (TRANSGENE) * Revendications 1-21 * | 1-12 | |
| Y | PROC. NATL. ACAD. SCI. USA, vol. 79, décembre 1982, pages 7415-7419; M. MACKETT et al.: "Vaccinia virus: a selectable eukaryotic cloning and expression vector" * En entier * | 1-8 | |

--- -/-

Le présent rapport de recherche a été établi pour toutes les revendications

| Lieu de la recherche | Date d'achèvement de la recherche | Examinateur |
|---|---|---|
| LA HAYE | 17-09-1987 | MADDOX A.D. |

**Office européen des brevets**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | J. CLIN. INVEST., vol. 76, décembre 1985, pages 635-637, The American Society for Clinical Investigation, Inc.; M. SCHAPIRA et al.: "Recombinant alphal-antitrypsin Pittsburgh (Met358-Arg) is a potent inhibitor of plasma kallikrein and activated factor XII fragment" * En entier * | 11,12 | |
| X,Y | DNA, vol. 5, no. 2, 1986, pages 129-136, Mary Ann Liebert Inc.; A. VAN DER STRATEN et al.: "Expression of cloned human haptoglobin and alphal-antitrypsin complementary DNAs in Saccharomyces cerevisiae" * Page 135, colonne de gauche * | 2 | |
| A | EP-A-0 110 385 (UNITED STATES DEPARTMENT OF COMMERCE) * En entier * | 1-12 | |
| A | EP-A-0 151 102 (SMITH KLINE) * Page 12, ligne 14 - page 13, ligne 20; page 23, lignes 5-28 * | 1-12 | |
| | --- -/- | | |

DOMAINES TECHNIQUES RECHERCHES (Int. Cl.4)

Le présent rapport de recherche a été établi pour toutes les revendications

| Lieu de la recherche | Date d'achèvement de la recherche | Examinateur |
|---|---|---|
| LA HAYE | 17-09-1987 | MADDOX A.D. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82

## DOCUMENTS CONSIDERES COMME PERTINENTS

Page 3

| Catégorie | Citation du document avec indication, en cas de besoin, des parties pertinentes | Revendication concernée | CLASSEMENT DE LA DEMANDE (Int. Cl.4) |
|---|---|---|---|
| A | BIOCHEMICAL AND BIOPHYSICAL RESEARCH COMMUNICATIONS, vol. 126, no. 1, 16 janvier 1985, pages 630-635, Academic Press Inc.; W. WEBER et al.: "Unglycosylated rat alpha1-proteinase inhibitor has a six-fold shorter plasma half-life than the mature glycoprotein" * En entier * | 9-12 | |
| A | FR-A-2 563 434 (TRANSGENE) * Page 16, ligne 25 - page 17, ligne 17; revendications 1-11 * | 1-12 | |
| A | EP-A-0 162 782 (TRANSGENE) * Page 16, lignes 1-22; page 19, ligne 7 - page 20, ligne 18; revendications 1-14 * & WO-A-85 05 376 (Cat. D) | 1-12 | DOMAINES TECHNIQUES RECHERCHES (Int. Cl.4) |
| A | J. INHERITED METAB. DIS., vol. 9, supplément 1, 1986, pages 85-91; F.D. LEDLEY et al.: "Molecular basis of alpha1-antitrypsin deficiency and its potential therapy by gene transfer" * En entier * | 1-12 | |

-/-

Le présent rapport de recherche a été établi pour toutes les revendications

| Lieu de la recherche<br>LA HAYE | Date d'achèvement de la recherche<br>17-09-1987 | Examinateur<br>MADDOX A.D. |
|---|---|---|

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

OEB Form 1503 03 82

Office européen
des brevets

**RAPPORT DE RECHERCHE EUROPEENNE**

Numéro de la demande

EP  87 40 1506

Page  4

**DOCUMENTS CONSIDERES COMME PERTINENTS**

| Catégorie | Citation du document avec indication, en cas de besoin, des parties pertinentes | Revendication concernée | CLASSEMENT DE LA DEMANDE (Int. Cl.4) |
|---|---|---|---|
| A | BIOLOGICAL ABSTRACTS/RRM, résumé no. 32031426, Philadelphia, US; J.L. DEMAYO et al.: "Expression of normal and mutant human alpha antitrypsin genes in transgenic mice", & AMERICAN JOURNAL OF HUMAN GENETICS(US), 1986, vol. 39, no. 3, suppl. pA195 | 1-12 | |
| | ----- | | |

DOMAINES TECHNIQUES
RECHERCHES (Int. Cl.4)

Le présent rapport de recherche a été établi pour toutes les revendications

| Lieu de la recherche | Date d'achèvement de la recherche | Examinateur |
|---|---|---|
| LA HAYE | 17-09-1987 | MADDOX A.D. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82